# EUROPÄISCHE PATENTSCHRIFT

(11) **EP 2 177 244 B1**
(45) Veröffentlichungstag und Bekanntmachung des Hinweises auf die Patenterteilung: **13.04.2016**
(21) Anmeldenummer: 08166918.6
(22) Anmeldetag: 17.10.2008
(51) Int. Cl.: A61N 5/10

(54) **Anlage zur Bestrahlung von Patienten mit geladenen Teilchen und Verfahren zur Überwachung der Anlage**
Assembly for irradiating patients with loaded particles and method for monitoring the assembly
Installation de rayonnement de patients dotée de particules chargées et procédé de surveillance de l'installation

(43) Veröffentlichungstag der Anmeldung: 21.04.2010
(73) Patentinhaber: AD Verwaltungs-GmbH & Co. KG, 82031 Grünwald (DE)
(72) Erfinder: Rinecker, Hans, 81379 München (DE); Hauffe, Jörg, 81541 München (DE)
(74) Vertreter: Kroher, Jürgen

(56) Entgegenhaltungen:
- EP-A- 0 978 294
- EP-A- 1 625 876
- WO-A-00/48678
- US-B1- 6 265 837
- KUTCHER G J ET AL: "COMPREHENSIVE QA FOR RADIATION ONCOLOGY: REPORT OF AAPM RADIATION THERAPY COMMITTEE TASK GROUP 40" MEDICAL PHYSICS, AIP, MELVILLE, NY, US, Bd. 21, Nr. 4, 1. April 1994 (1994-04-01), Seiten 581-618, XP000953664 ISSN: 0094-2405
- JAEKEL O ET AL: "QUALITY ASSURANCE FOR A TREATMENT PLANNING SYSTEM IN SCANNED ION BEAM THERAPY" MEDICAL PHYSICS, AIP, MELVILLE, NY, US, Bd. 27, Nr. 7, 1. Juli 2000 (2000-07-01), Seiten 1588-1600, XP000936499 ISSN: 0094-2405
- FATEMEH AZMANDIAN ET AL: "Towards the development of an error checker for radiotherapy treatment plans: a preliminary study" PHYSICS IN MEDICINE AND BIOLOGY, TAYLOR AND FRANCIS LTD. LONDON, GB, Bd. 52, Nr. 21, 7. November 2007 (2007-11-07), Seiten 6511-6524, XP020127240 ISSN: 0031-9155

## Beschreibung

Die vorliegende Erfindung betrifft eine Anlage zur Bestrahlung von Patienten mit geladenen Teilchen und ein Verfahren zur Überwachung der Anlage.

Derartige Anlagen besitzen im Vergleich zu konventionellen Bestrahlungsanlagen, die mit Röntgen- bzw. Photonenstrahlen arbeiten, erhebliche Vorteile hinsichtlich der Zielgenauigkeit der Dosisapplikation sowie der Verringerung der Nebenwirkungen im gesunden Gewebe.

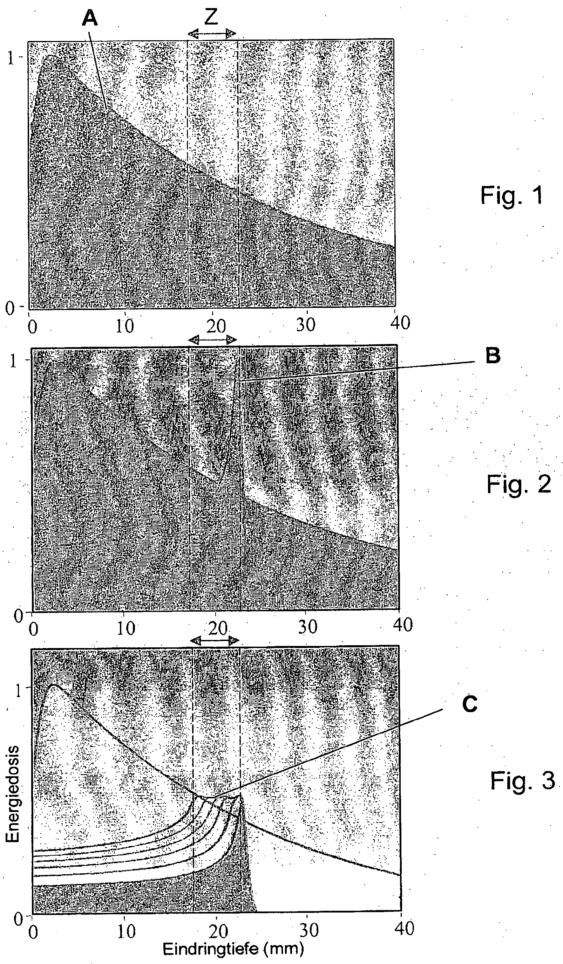

Konventionelle Photonenstrahlen durchdringen den Körper, werden aber auch bei ihrer Interaktion mit den Molekülen des Körpers absorbiert und verlieren dadurch stetig an Intensität. Das Dosismaximum liegt dabei knapp unter der Haut, wie aus der Dosisverteilungskurve A aus Fig. 1 ersichtlich ist. Dieser Effekt beruht auf der erst unmittelbar unter der Haut eintretenden Rekrutierung von Streustrahlung. Auf dem weiteren Weg in Richtung des Tumorbereichs Z fällt die Strahlendosis dann in Form einer Exponentialkurve ab. Ein tief im Körper liegender Tumor erhält somit weniger Dosis als gesundes Gewebe im Strahlengang vor ihm, während hinter dem Tumor liegende Organe immer noch eine beträchtliche Dosis erhalten.

Im Gegensatz hierzu geben geladene Teilchen wie Protonen und Schwerionen nach dem Eindringen in den Körper zunächst nur relativ wenig Energie ab, werden aber durch wiederholte Wechselwirkung mit Materie gebremst (siehe Fig. 2). Je langsamer die Partikel werden, desto höher ist die abgegebene Energie und die Abbremsung. Dies führt zu einer "Energieexplosion" am Ende der Teilchenbahn, dem sog. "Bragg-Peak" (Dosisverteilungskurve B in Fig. 2). Vor dem Tumor ist im Gegensatz zur Bestrahlung mit Photonen bei geladenen Teilchen die Dosis wesentlich niedriger bei gleichzeitiger Konzentration der Dosis in den Tumor. Bei Protonen bleibt der Patient hinter dem Tumor sogar strahlungsfrei. Dieses physikalische Phänomen erlaubt durch Beeinflussung der erzeugten Partikelgeschwindigkeit in Verbindung mit dem Scanningverfahren, die Dosis dreidimensional in den Tumor zu zielen. Der Bragg-Peak ist so scharf, dass er nicht nur seitlich, sondern durch Variation der Teilchengeschwindigkeit auch in der Tiefe über den Tumor hinweg verfahren werden muss, wie aus Fig. 3 ersichtlich ist, in der ein Bragg-Plateau C dargestellt ist.

Ein bevorzugtes Verfahren, bei dem der Bragg-Peak des Strahls geladener Teilchen den Tumor millimetergenau in einem zuvor durch Diagnostik und Bestrahlungsplanung festgelegten dreidimensionalen Raster computergesteuert abfährt, ist das sog. Rasterscan-Verfahren. Bei diesem Verfahren beträgt der Strahldurchmesser typischerweise 10 mm FWHM, und die einzelnen Rasterpunkte werden nacheinander angesteuert und in jeder Bestrahlungssitzung in typischerweise 60 bis 90 Sekunden mit der jeweils festgelegten Dosis bestrahlt. Eine Patientenbehandlung erfolgt in mehreren Bestrahlungssitzungen an aufeinander folgenden Tagen.

Insbesondere bei nahe an lebenswichtigen gesunden Strukturen liegenden Tumoren und sehr tief liegenden Tumoren, die man aufgrund der zwangsläufigen unerwünschten Schädigung des gesunden Gewebes in vielen Fällen überhaupt nicht, in anderen Fällen nur mit hohem Risiko mit herkömmlicher Photonenstrahlentherapie behandeln kann, stellt die Bestrahlung mit geladenen Teilchen einen erheblichen Fortschritt in der Krebsbehandlung dar.

Beim Bestrahlen mit geladenen Teilchen nach dem Rasterscan-Verfahren bringt die oben erläuterte scharfe und konzentrierte Dosisverteilung des Bleistift-Strahls und die damit verbundene zielgenaue dreidimensionale Bestrahlung auch erhöhte Anforderungen an die einzuhaltende Genauigkeit mit sich.

Hierzu bedarf es einer höchst exakten Therapieplanung. Außerdem ist es beispielsweise aus EP 1 625 876 A1 bekannt, nach einer oder mehreren Bestrahlungen eine weitere Bildaufnahme des zu bestrahlenden Gewebes zu machen, um festzustellen, ob eventuell eine Abweichung der neuen Aufnahme von der ursprünglich dem Therapieplan zugrundeliegenden Aufnahme vorhanden ist, beispielsweise hinsichtlich relevanter geometrischer Informationen wie Form und Lage des Tumors oder Volumen und durchstrahlte Weglänge in Einstrahlrichtung zwischen Haut und Zielgebiet. Unter Umständen soll hieraus sogar ein automatischer Planungshinweis für eine geänderte Parametereinstellung der Bestrahlungseinheit erzeugt werden können.

Außerdem umfassen Anlagen zur Bestrahlung von Patienten mit geladenen Teilchen eine Mehrzahl von Sicherheitseinrichtungen zum Überprüfen der Therapieplanungsdaten und der Funktionalität der Anlage, mit der eine falsche Berechnung der Therapieplanungsdaten und Datenübertragungsfehler zwischen den einzelnen Komponenten der Anlage minimiert werden können. Damit kann auch eine punktuelle Datenkorruption (random errors) noch sicherer ausgeschlossen werden, die sich ebenfalls sehr nachteilig auf den Bestrahlungserfolg auswirken kann.

Derartige Sicherheitseinrichtungen sind beispielsweise aus dem nächstkommenden Stand der Technik WO 00/48678 A1 bekannt. Unter anderem ist hierbei vorgesehen, Programme und Daten in die einzelnen Steuerungsrechner der Bestrahlungseinheit zu schreiben, zurückzulesen und mit in Speichern des Kontrollsystems gespeicherten Daten und Programmen zu vergleichen, um eine systemimmanente Datenkorruption bei der Übertragung der erzeugten Therapieplanungsdaten und -befehle auf dem Weg vom Kontrollsystem zu den Steuerungsrechnern der Bestrahlungseinheit auszuschließen. Allerdings erfolgt hierbei lediglich ein Vergleich der empfangenen mit den gesendeten Daten, die Therapieplanungsdaten und -befehle werden aber nicht mehr auf klinisch relevante Aspekte überprüft.

Der vorliegenden Erfindung liegt daher die Aufgabe zugrunde, eine Anlage zur Bestrahlung von Patienten mit geladenen Teilchen zu schaffen, die eine weitere besonders effiziente Redundanz hinsichtlich der Sicherheitsvorkehrungen schafft, die dem klinischen Anwender einen transparenten Zugang zu den überwachten Daten ermöglicht und mit der auch das Risiko einer punktuellen Datenkorruption von der Behandlung zugrunde liegenden Daten unmittelbar vor der Bestrahlung minimiert werden kann, sowie ein entsprechendes Verfah-ren zur Überwachung der Anlage zur Bestrahlung von Patienten mit geladenen Teilchen anzugeben.

Diese Aufgabe wird durch die Merkmale des Anspruchs 1 bzw. des Anspruchs 5 gelöst.

Erfindungsgemäß weist die Anlage zur Bestrahlung von Patienten mit geladenen Teilchen eine Raster-Scanning-Bestrahlungseinheit auf, die einen Teilchenbeschleuniger, eine Strahlführungseinheit und ein 3D-Scanning-System aufweist, wobei das 3D-Scanning-System eine Energievariationseinheit zur Einstellung der Energie des Teilchenstrahls und damit der Eindringtiefe des Strahls in den Patienten in Strahlrichtung und eine Ablenkeinheit mit mehreren Ablenkmagneten zum zweidimensionalen Ablenken des Strahls zwischen einzelnen Rasterpunkten in jeder durch die Energievariationseinheit definierten, quer zur Strahlrichtung angeordneten Schicht vorgegebener Eindringtiefe im Patienten aufweist. Außerdem weist die Anlage ein Therapieplanungssystem zum Erzeugen von Therapieplanungsdaten auf, welche die aus der gewünschten Dosisverteilung abgeleitete Energie und Anzahl von geladenen Teilchen pro Rasterpunkt in jeder Schicht umfassen, sowie ein Therapiekontrollsystem, das die vom Therapieplanungssystem erzeugten Therapieplanungsdaten in Bestrahlungsdaten und Bestrahlungsbefehle für den Teilchenbeschleuniger, die Strahlführungseinheit und das 3D-Scanning-System umwandelt. Schließlich weist die Anlage eine Mehrzahl von Sicherheitseinrichtungen zum Überprüfen der korrekten Umsetzung der Therapieplanungsdaten und der Funktionalität der Anlage auf, die eine Auswerteeinheit umfassen, welche die vom Therapiekontrollsystem an das 3D-Scanning-System gelieferten Bestrahlungsdaten und Bestrahlungsbefehle auf ihre therapiespezifische Plausibilität überprüft.

Das entsprechende Verfahren zur Überwachung einer Anlage zur Bestrahlung von Patienten mit geladenen Teilchen weist folgende Schritte auf:
Bereitstellen einer Raster-Scanning-Bestrahlungseinheit, die einen Teilchenbeschleuniger, eine Strahlführungseinheit und ein 3D-Scanning-System aufweist, wobei das 3D-Scanning-System eine Energievariationseinheit zur Einstellung der Energie des Teilchenstrahls und damit der Eindringtiefe des Strahls in den Patienten in Strahlrichtung und eine Ablenkeinheit mit mehreren Ablenkmagneten zum zweidimensionalen Ablenken des Strahls zwischen einzelnen Rasterpunkten in jeder durch die Energievariationseinheit definierten, quer zur Strahlrichtung angeordneten Schicht vorgegebener Eindringtiefe im Patienten aufweist;
Erzeugen von Therapieplanungsdaten in einem Therapieplanungssystem, wobei die Therapieplanungsdaten die aus der gewünschten Dosisverteilung abgeleitete Energie und Anzahl von geladenen Teilchen pro Rasterpunkt in jeder Schicht umfassen;
Umwandeln der vom Therapieplanungssystem erzeugten Therapieplanungsdaten in Bestrahlungsdaten und Bestrahlungsbefehle für den Teilchenbeschleuniger, die Strahlführungseinheit und das 3D-Scanning-System mittels eines Therapiekontrollsystems zur Bestrahlung des Patienten; und
Überprüfen der korrekten Umsetzung der Therapieplanungsdaten und der Funktionalität der Anlage mittels einer Mehrzahl von Sicherheitseinrichtungen. Dabei umfasst das Überprüfen der korrekten Umsetzung der Therapieplanungsdaten und der Funktionalität der Anlage das Überprüfen der vom Therapiekontrollsystem an das 3D-Scanning-System gelieferten Bestrahlungsdaten und Bestrahlungsbefehle mittels einer Auswerteeinheit auf ihre therapiespezifische Plausibilität.

In einem ersten Untersuchungsverfahren überprüft die Auswerteeinheit die vom Therapiekontrollsystem erzeugte Datei mit Bestrahlungsdaten und Bestrahlungsbefehlen auf ihre therapiespezifische Plausibilität hinsichtlich erlaubter Energiebereiche der geladenen Teilchen. Diese Energiebereiche werden entweder im Vorfeld festgelegt oder durch die Therapieplanungsdaten bestimmt. Dadurch werden unerlaubte Energiebereiche für die Applikation ausgeschlossen.

Vorzugsweise ist die Raster-Scanning-Bestrahlungseinheit mit einem drehbaren Gantry ausgestattet, und die Auswerteeinheit überprüft die vom Therapiekontrollsystem erzeugte Datei mit Bestrahlungsdaten und Bestrahlungsbefehlen auf ihre therapiespezifische Plausibilität hinsichtlich erlaubter Gantry-Winkel für die Applikation der geladenen Teilchen. Damit kann auf einfache Weise eine falsche Einstellung von Gantry-Winkeln verhindert werden. Die erlaubten Gantry-Winkel können dabei sowohl durch anwenderspezifische Vorgaben als auch durch einen mittels der Therapieplanung vorgegebenen Bereich definiert werden.

In einer weiteren Überprüfungsstufe überprüft die Auswerteeinheit die vom Therapiekontrollsystem erzeugte Datei mit Bestrahlungsdaten und Bestrahlungsbefehlen auf ihre therapiespezifische Plausibilität hinsichtlich der Anzahl von zu applizierenden geladen Teilchen pro Rasterpunkt.

Dabei kann die Zahl von geladenen Teilchen pro Rasterpunkt mit einem vorgegebenen maximalen Grenzwert, welcher nicht überschritten werden darf, verglichen werden. Ebenso kann der Durchschnitt der Anzahl von geladenen Teilchen pro Rasterpunkt mit üblichen Werten verglichen werden. Auf diese Weise wird eine Überdosis für einzelne Rasterpunkte in letztinstanzlicher Kontrolle ausgeschlossen.

Der Bestrahlungsablauf erfolgt immer in einer vorbestimmten Abfolge, welche nach logischen Ordnungsprinzipien vorgegeben ist. Vorzugsweise untersucht die Auswerteeinheit die vom Therapiekontrollsystem erzeugte Datei mit Bestrahlungsdaten und Bestrahlungsbefehlen auch auf ihre therapiespezifische Plausibilität hinsichtlich dieser vorbestimmter logischen Ordnungsprinzipien.

Darunter fällt beispielsweise die streng abnehmende Monotonität der Energiewerte der zu applizierenden geladenen Teilchen zwischen den einzelnen Bestrahlungsschichten, welche der abnehmenden Eindringtiefe zwischen den einzelnen beim Scanning zu durchlaufenden Schichten entspricht. Die streng abnehmende Monotonität der Energiewerte der zu applizierenden geladenen Teilchen kann mittels der Auswerteeinheit überprüft werden. Durch eine solche Überprüfung können Fehler bei der Eindringtiefe des Teilchenstrahls für jede Schicht ausgeschlossen werden.

Ebenfalls in diese Kategorie fällt die Überprüfung der Reihenfolge und Anordnung der beim Scanning zu durchlaufenden Rasterpunkte für jede Schicht mittels der Auswerteeinheit. Dabei leitet die Auswerteeinheit die x-Werte und y-Werte für alle Rasterpunkte einer Schicht vorgegebener Eindringtiefe ab, ermittelt daraus den Verlauf der zweidimensionalen Anordnung der Rasterpunkte in der Reihenfolge der Applikation und überprüft diesen Verlauf auf vorbestimmte Kriterien. Solche Kriterien sind beispielsweise das Vorliegen von Rasterpunkten, die nicht auf dem vorgegebenen Raster liegen, zu große Sprünge in x-Richtung oder y-Richtung usw. Mittels dieser Überprüfung können einzelne Ausreißer auf einfache Weise detektiert und ausgeschlossen werden.

Hierdurch wird unmittelbar vor der Bestrahlung eine zusätzliche Sicherheitskomponente instrumentalisiert, die sogar eine punktuelle Datenkorruption in den Bestrahlungsdaten und Bestrahlungsbefehlen erkennen kann und somit die Sicherheit der Anlage noch weiter erhöht. Dabei werden insbesondere die klinisch bestimmten Zusammenhänge herangezogen, um eine schnelle und effiziente Plausibilitätsprüfung durchzuführen und damit die Sicherheit zu erhöhen.

Vorzugsweise ist die Auswerteeinheit entweder in einem Scanning-Steuermodul angeordnet, das einen Teil des 3D-Scanning-Systems bildet und dazu geeignet ist, die vom Therapiekontrollsystem versandten Bestrahlungsdaten und Bestrahlungsbefehle entgegenzunehmen, oder sie ist zwischen Therapiekontrollsystem und dem Scanning-Steuermodul angeordnet. Die Auswerteeinheit untersucht eine vom Therapiekontrollsystem erzeugte Datei mit Bestrahlungsdaten und Bestrahlungsbefehlen auf ihre therapiespezifische Plausibilität und erzeugt eine Nachricht über das Ergebnis der Untersuchung. Durch die unmittelbare Vorlagerung der Auswerteeinheit vor der tatsächlichen Teilchenapplikation wird eine direkte und sichere Endkontrolle der an das 3D-Scanning-System gelieferten Bestrahlungsdaten und Bestrahlungsbefehle auf ihre therapiespezifische Sinnhaftigkeit hin ermöglicht.

Die x-Werte und y-Werte für alle Rasterpunkte leitet die Auswerteeinheit vorzugsweise aus den Magnetströmen der Ablenkmagnete der Ablenkeinheit ab.

Besonders bevorzugt ist eine weitere Überprüfungsform, nach der alle vom Therapiekontrollsystem an das 3D-Scanning-System gelieferten Bestrahlungsdaten und Bestrahlungsbefehle für eine Bestrahlungssitzung in der Auswerteeinheit gespeichert werden und am nächsten Tag mit den gesamten dann erneut vom Therapiekontrollsystem an das 3D-Scanning-System gelieferten Bestrahlungsdaten und Bestrahlungsbefehlen für die nächste Bestrahlungssitzung verglichen werden. Da die Therapiepläne üblicherweise vollkommen identische Bestrahlungspläne für sämtliche Sitzungen vorsehen, können auf diese Weise besonders einfach Abweichungen festgestellt werden, die in erster Linie aufgrund geänderter äußerer Einflüsse wie Temperatur, Luftdruck, Konfigurationsparameter der Anlage usw. auftreten können.

Die bislang vorgestellten Überprüfungsverfahren haben den besonderen Vorteil, dass sie wenig rechen- und zeitintensiv sind und somit eine unmittelbare Überprüfung der vom Therapiekontrollsystem an das 3D-Scanning-System gelieferten Bestrahlungsdaten und Bestrahlungsbefehle ermöglichen. In einer weitergehenden Anwendung kann die Auswerteeinheit aus der Anzahl und Energie der auf jeden Rasterpunkt zu applizierenden geladenen Teilchen durch eine Rückrechnung eine Raster-Dosisverteilung berechnen und diese nach vorgegebenen Kriterien auf ihre therapiespezifische Plausibilität hin überprüfen. Der hiermit verbundene Rechenaufwand ist erheblich, jedoch können dadurch Löcher und Inseln in der Dosisverteilung des dreidimensionalen Tumors sehr genau ermittelt und ausgeschlossen werden.

Weitere Eigenschaften, Merkmale und Vorteile der vorliegenden Erfindung ergeben sich aus der nachfolgenden Beschreibung unter Bezugnahme auf die Zeichnungen:
- Fig. 1: zeigt eine Dosisverteilungskurve bei der konventionellen Bestrahlung mit Photonen;
- Fig. 2: zeigt eine Dosisverteilungskurve bei der Bestrahlung mit Protonen einer bestimmten Energie im Vergleich zur Dosisverteilungskurve bei der Bestrahlung mit Photonen;
- Fig. 3: zeigt die Überlagerung verschiedener Dosisverteilungskurven bei der Bestrahlung mit Protonen zur Abtastung des Tumors;
- Fig. 4: zeigt den schematischen Aufbau einer Anlage zur Bestrahlung von Patienten mit geladenen Teilchen;
- Fig. 5: zeigt eine Detaildarstellung eines Ausführungsbeispiels des 3D-Scanning-Systems;
- Fig. 6: zeigt eine schematische Übersicht über die elektronischen Komponenten einer erfindungsgemäßen Anlage, welche an der Berechnung und Übertragung von Bestrahlungsdaten und Bestrahlungsbefehlen beteiligt sind;
- Fig. 7: zeigt Auszüge des Inhalts einer Beispieldatei, welche vom Therapiekontrollsystem an das 3D-Scanning-System zu überliefernde Bestrahlungsdaten und Bestrahlungsbefehle aufweist;
- Fig. 8: zeigt eine Graphik zur Überprüfung der Bestrahlungsdaten hinsichtlich erlaubter Energiebereiche der geladenen Teilchen in verschiedenen Schichten von Bestrahlungstiefe;
- Fig. 9: zeigt eine Graphik zur Überprüfung der Bestrahlungsdaten hinsichtlich erlaubter Gantry-Winkel;
- Fig. 10: zeigt eine Graphik zur Überprüfung der Bestrahlungsdaten hinsichtlich der Anzahl von zu applizierenden geladenen Teilchen pro Rasterpunkt; und
- Fig. 11: zeigt graphische Darstellungen zur Überprüfung der Bestrahlungsdaten bezüglich der Reihenfolge und Anordnung der beim Scanning zu durchlaufenden Rasterpunkte für jede Schicht in verschiedenen Darstellungsformen.

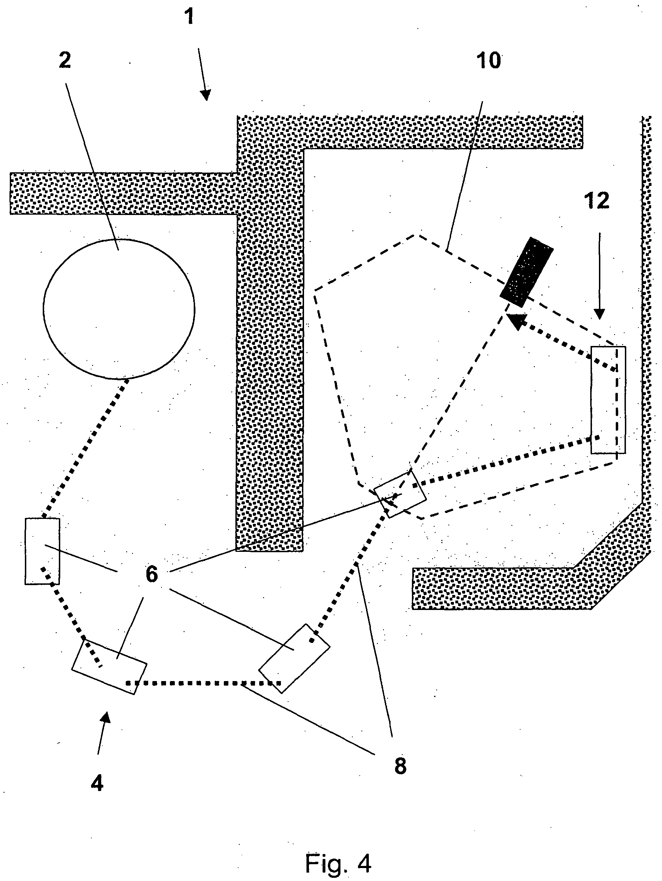

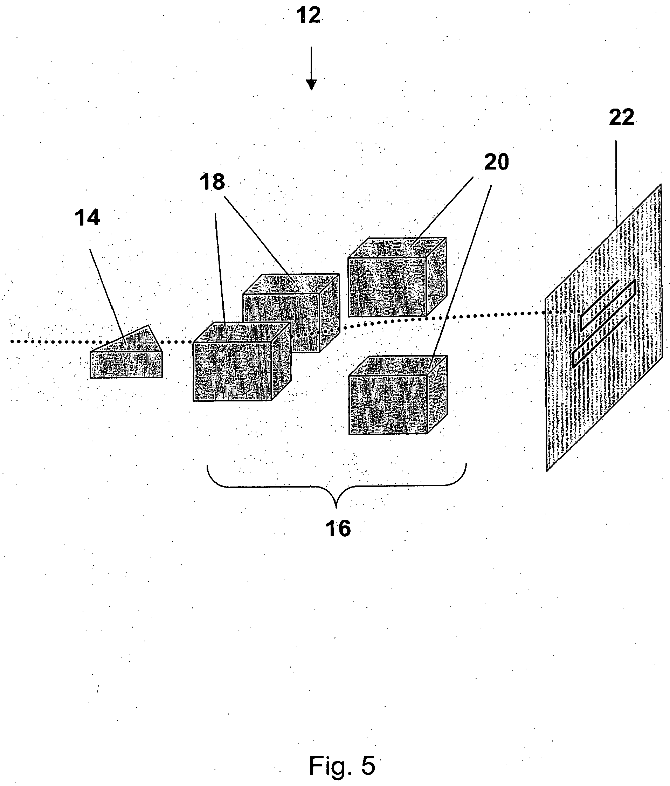

Um das in Fig. 3 dargestellte gleichmäßige oder auf gewünschte Weise anpassbare Bestrahlungsprofil eines Tumorgewebes im menschlichen Körper zu erzielen, umfasst die erfindungsgemäße Anlage zur Bestrahlung von Patienten mit geladenen Teilchen eine Raster-Scanning-Bestrahlungseinheit 1, die schematisch in Fig. 4 dargestellt ist. Die Raster-Scanning-Bestrahlungseinheit 1 weist einen Teilchenbeschleuniger 2 für geladene Teilchen auf. Als geladene Teilchen zur Bestrahlung von Tumoren kommen beispielsweise Protonen oder Schwerionen in Frage. Die Raster-Scanning-Bestrahlungseinheit 1 weist außerdem eine Strahlführungseinheit 4 auf, die aus mehreren Strahlführungsmagneten 6 und zwischen diesen angeordneten, meist gerade verlaufenden Strahlführungsabschnitten 8 besteht. Die exakte Strahlführung ist eine der wichtigsten Herausforderungen bei der Bestrahlung mit geladenen Teilchen. Der Teilchenstrahl wird von der Strahlführungseinheit 4 in einen Behandlungsraum geleitet, in dem im vorliegenden Beispielsfall ein Gantry 10 angeordnet ist, welches sich um 360° drehen lässt. Das 3D-Scanning-System 12 dient zur zielgenauen Bestrahlung des Tumorgewebes und weist verschiedene, weiter unten in Bezugnahme auf Fig. 5 näher beschriebene Elemente zur Detailsteuerung des Teilchenstrahls auf.

Im dargestellten Beispielsfall ist die Ablenkeinheit 14 des 3D-Scanning-Systems 12 mit dem Ring des Gantrys 10 verbunden und kann gemeinsam mit diesem in eine beliebige Position gedreht werden, aus der der Patient bestrahlt wird. Hierdurch ist eine Bestrahlung aus verschiedenen Richtungen für verschiedene Applikationen möglich. Ebenso ist die Erfindung auf stationäre Bestrahlungsapparate anwendbar, die nicht als Gantry 10 ausgebildet sind.

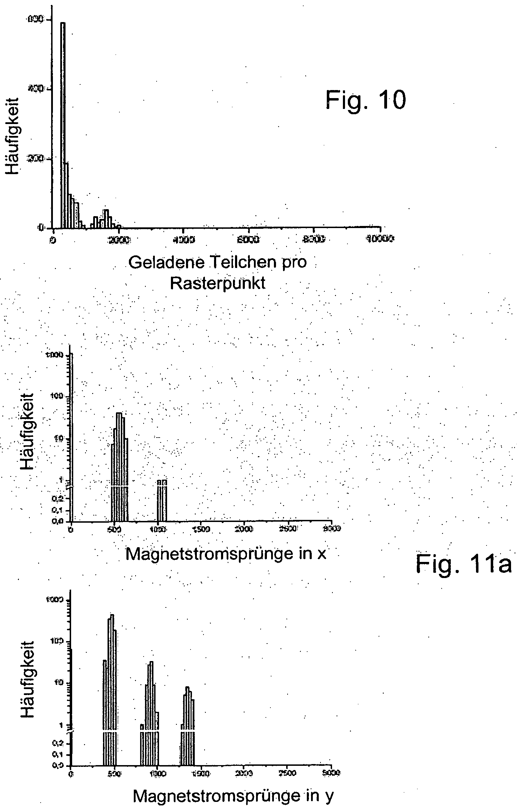

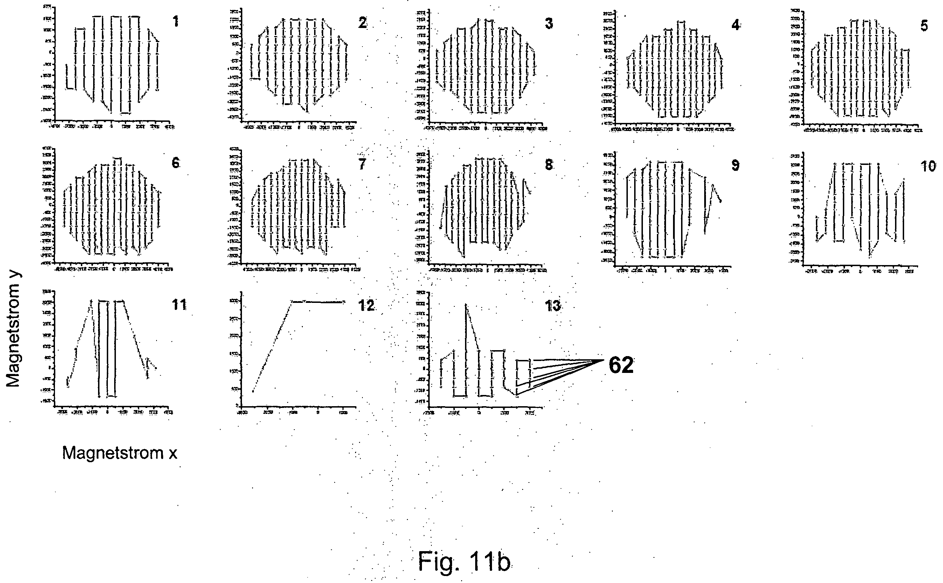

Mit der erfindungsgemäßen Anlage werden die Patienten vorzugsweise in einem RasterScan-Verfahren bestrahlt, bei dem das Tumorgewebe in gleichmäßig beabstandete Rasterpunkte 62 (siehe Fig. 11 b) in einem dreidimensionalen Raster unterteilt wird, an denen eine Dosisapplikation stattfindet.

Um den Tumor auf gezielte Weise mit dem Behandlungsstrahl abzutasten bzw. zur genauen Ansteuerung der einzelnen 3D-Rasterpunkte 62 im Zielvolumen besitzt das 3D-Scanning-System 12 (siehe Fig. 5) zunächst eine Energievariationseinheit 14 zur Einstellung der Energie des Teilchenstrahls und damit der Eindringtiefe des Strahls in den Patienten in Strahlrichtung. Im vorliegenden Beispiel ist die Energievariationseinheit 14 als Degrader-Keil ausgebildet, der jeweils über eine bestimmte Strecke in den Strahlengang geschoben werden kann und somit einen bestimmten Anteil der Energie des Teilchenstrahls absorbiert. Auf diese Weise ist die Energie des Teilchenstrahls und somit die Eindringtiefe des Teilchenstrahls in Strahlrichtung in den Körper submillimetergenau zu bestimmen. Neben einem Degrader-Keil sind auch andere Energievariationseinheiten 14 denkbar, beispielsweise Range Shifter-Platten.

Innerhalb einer jeden durch die Energievariationseinheit 14 definierten, quer zur Strahlrichtung angeordneten Schicht 22 vorgegebener Eindringtiefe im Patienten kann der Teilchenstrahl mittels einer Ablenkeinheit 16 gesteuert werden. Die Ablenkeinheit 16 weist beispielsweise einen zweipoligen Ablenkmagneten 18 zur Ablenkung des Teilchenstrahls in x-Richtung und einen zweipoligen Ablenkmagneten 20 zur Ablenkung des Teilchenstrahls in y-Richtung auf. Auch andere Ausgestaltungen sind denkbar. Die Ablenkeinheit 16 erzeugt somit in jeder Schicht 22 vorbestimmter Eindringtiefe ein mäanderndes Profil des Teilchenstrahls, wobei der Teilchenstrahl jeweils zwischen zwei Rasterpunkten 62 ausgeschaltet bleibt und erst nach vollständiger Einstellung der Ablenkmagnete 18, 20 auf den Tumor gelenkt wird, um eine präzise Dosisapplikation zu gewährleisten. Die Dosis bzw. die Anzahl von zu applizierenden geladenen Teilchen an benachbarten Rasterpunkten 62 kann sich dabei je nach Form des Tumors erheblich unterscheiden.

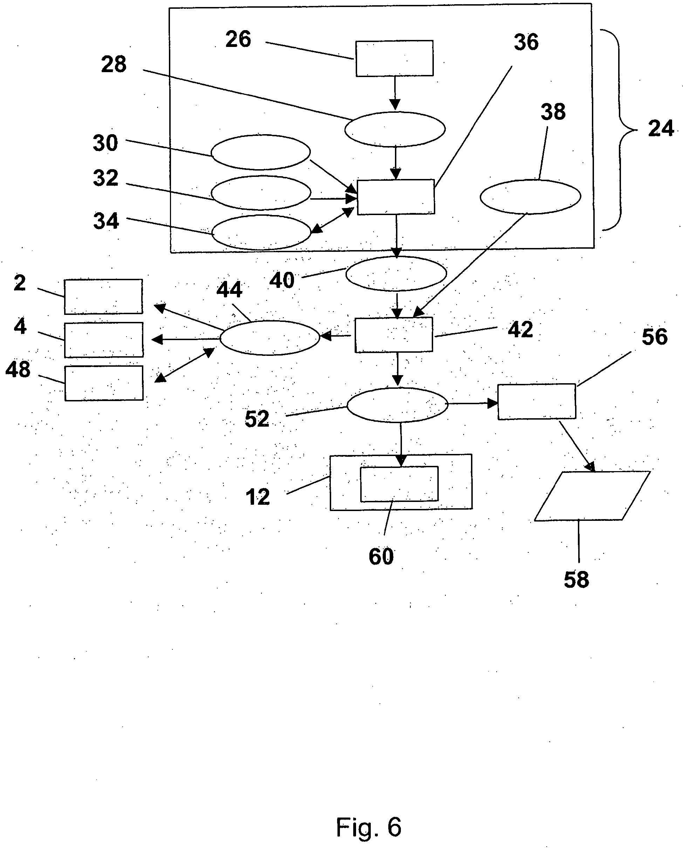

Für die Therapieplanung und die exakte Bestrahlung ist ein hochkomplexes System vorgesehen, welches die einzelnen Bestrahlungsparameter bis ins Kleinste festlegt und die Funktionalität der Anlage überwacht. Die elektronischen Komponenten einer erfindungsgemäßen Anlage, die an der Berechnung und Übertragung von Bestrahlungsdaten und Bestrahlungsbefehlen beteiligt sind, sind in Fig. 6 dargestellt. Das Therapieplanungssystem 24 umfasst eine Therapieplanungsstufe 26 zur Dosisberechnung und -optimierung. Darin fließen alle Ergebnisse der vorab durchgeführten medizinischen Beurteilung, Indikationsstellung, Zielvolumenkonturierung und des Therapiekonzepts sowie auch die dazugehörigen CT-Daten ein. Die Therapieplanungsstufe 26 berechnet daraus zunächst die gewünschte Dosisverteilung {x, y, z, Dosis} unter Berücksichtigung der vom zuständigen Arzt festgesetzten Parameter. Anschließend wird unter Einbeziehung der Funktionsdaten der Gesamtanlage daraus ein Therapieplan 28 generiert, der die aus der gewünschten Dosisverteilung berechnete Energie und Anzahl von geladenen Teilchen pro Rasterpunkt 62 in jeder Schicht 22 festlegt. Somit wird in der so genannten Spotwelt jedem Rasterpunkt 62 eine Information der Art {x, y, Energie der Teilchen, Anzahl der Teilchen} zugeordnet. Diese Daten des Therapieplans 28 werden an einen Therapiedatenspeicher 36 übermittelt, welcher zudem alle Patientendaten 30 und die CT-Daten 32, beispielsweise für die Positionsverifikation, enthält. Außerdem sind an dieser Stelle bereits Sicherheitseinrichtungen 34 vorgesehen, welche den Therapieplan genau überprüfen.

Das Therapieplanungssystem 24 stellt dann, beispielsweise aus dem Therapiedatenspeicher 36, alle Therapieplanungsdaten 40 dem Therapiekontrollsystem 42 bereit. Ebenso werden auch Konfigurationsdaten 38 der Anlage berücksichtigt, darunter bestimmte Maschineneinstellungen und -konfigurationen.

Das Therapiekontrollsystem 42 wandelt die vom Therapieplanungssystem 24 erzeugten Therapieplanungsdaten 40 in Bestrahlungsbefehle 44 für den Teilchenbeschleuniger 2 und die Strahlführungseinheit 4 um. Alle maschinenrelevanten Daten werden außerdem in hoch technisierten Sicherheitseinrichtungen 48 dauerhaft überprüft und überwacht. Dies reicht von Überwachung der Türen über die Strahlüberwachung bis hin zur Überprüfung der Sensoren (nicht dargestellt), welche den Behandlungsstrahl während der Bestrahlung überprüfen und an sich schon eine eigene weitere Sicherheitseinrichtung darstellen.

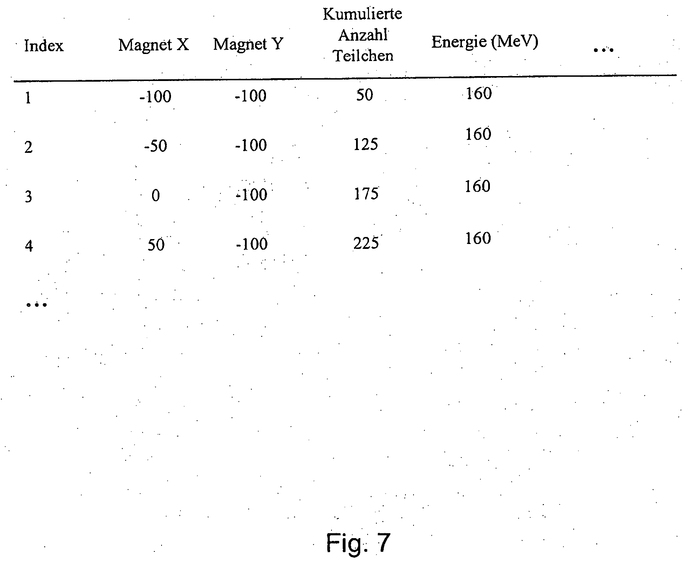

Außerdem wandelt das Therapiekontrollsystem 42 die vom Therapieplanungssystem 24 erzeugten Therapieplanungsdaten 40 auch in Bestrahlungsdaten und Bestrahlungsbefehle 52 für das 3D-Scanning-System 12 um. Vorzugsweise ist im 3D-Scanning-System 12 ein Scanning-Steuermodul 60 enthalten, welches dazu geeignet ist, die vom Therapiekontrollsystem versandten Bestrahlungsdaten und Bestrahlungsbefehle 52 entgegenzunehmen und die Ansteuerung der Bestandteile des 3D-Scanning-Systems 12 auf dieser Basis zu übernehmen. Zwischen dem Therapiekontrollsystem 42 und dem Scanning-Steuermodul 60 oder im Scanning-Steuermodul 60 selbst ist nun gemäß der Erfindung eine Auswerteeinheit 56 angeordnet, welche die vom Therapiekontrollsystem 42 an das 3D-Scanning-System 12 gelieferten Bestrahlungsdaten und Bestrahlungsbefehle 52 vor ihrer tatsächlichen Abarbeitung bzw. Umsetzung auf ihre therapiespezifische Plausibilität überprüft. Dies bedeutet, dass die übermittelten Bestrahlungsdaten und Bestrahlungsbefehle 52 in mindestens einer Hinsicht, vorzugsweise aber bezüglich mehrerer therapiespezifischer Parameter hin untersucht werden, die einen einfachen, aber umfassenden Plausibilitätscheck der Bestrahlungsdaten und Bestrahlungsbefehle 52 aus klinischer Sicht für den jeweiligen spezifischen Anwendungsfall gewährleisten. Hierauf wird weiter unten unter Bezugnahme auf Fig. 7 bis 11 noch näher eingegangen.

Vorzugsweise ist die Auswerteeinheit 56 dazu geeignet, eine Nachricht 58 über das Ergebnis der Untersuchung zu erzeugen, welche als Grundlage für die Unterbrechung der Behandlung oder andere Anpassungsmaßnahmen dienen kann.

Üblicherweise sind die vom Therapiekontrollsystem 42 an das Scanning-Steuermodul 60 übermittelten Bestrahlungsdaten und Bestrahlungsbefehle 52 in einer einzigen Datei enthalten, deren Inhalt beispielhaft in Fig. 7 wiedergegeben ist. Beispielsweise können die Bestrahlungsdaten und Bestrahlungsbefehle 52 den Magnetstrom in x-Richtung, den Magnetstrom in y-Richtung, die Anzahl der geladenen Teilchen pro Rasterpunkt 62 und die Energie des Teilchenstrahls enthalten sowie beispielsweise Überwachungsgrenzwerte der Detektoren des 3D-Scanning-Systems 12 und viele weitere bestrahlungsspezifische und relevante Parameter.

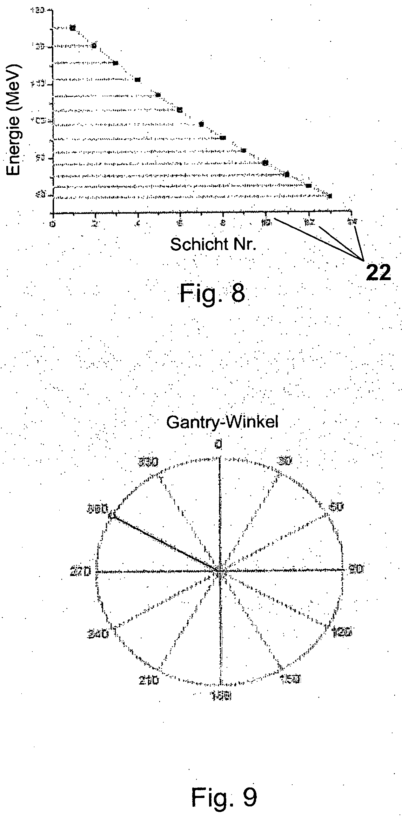

Die Auswerteeinheit 56 überprüft nun beispielsweise, wie in Fig. 8 dargestellt ist, die angeforderte Energie des Teilchenstrahls auf erlaubte Werte für die spezielle Behandlung. Zu hohe oder zu niedrige Teilchenenergien können damit jederzeit entdeckt werden. Auf ähnliche Weise kann der Gantry-Winkel (siehe Fig. 9) auf seine therapiespezifische Plausibilität für die Applikation der geladenen Teilchen im speziellen Anwendungsfall überprüft werden.

Ebenfalls hilfreich ist die Auswertung der Bestrahlungsdaten und Bestrahlungsbefehle 52 auf ihre therapiespezifische Plausibilität hinsichtlich der Anzahl von zu applizierenden geladenen Teilchen pro Rasterpunkt 62, wie in Fig. 10 dargestellt ist. Auch hier können bestimmte Unter- und Obergrenzen eingestellt werden, deren Einhaltung für den klinischen Anwender einfach zu überprüfen ist.

In Fig. 11a sind die Magnetstromsprünge in x-Richtung und in y-Richtung nach ihrer Häufigkeit aufgetragen. In einer solchen Darstellungsart erkennt man deutlich die Rastereinstellungen der gewünschten Bestrahlung und kann Abweichungen, z.B. zwischen den gewünschten Rastereinstellungen des Magnetstroms befindliche Magnetstromsprünge oder starke Ausreißer, besonders einfach auffinden.

Eine weitere Überprüfung der Bestrahlungsdaten und Bestrahlungsbefehle 52 auf ihre therapiespezifische Plausibilität hinsichtlich vorbestimmter logischer Ordnungsprinzipien liegt beispielsweise auch vor, wenn die streng abnehmende Monotonität der Energiewerte der zu applizierenden geladenen Teilchen überprüft wird, um die kontinuierlich abnehmende Eindringtiefe zwischen den einzelnen beim Scanning zu durchlaufenden Schichten 22 zu verifizieren.

Ebenso kann die Auswerteeinheit 56 in einer besonders bevorzugten Ausführungsform die Reihenfolge und Anordnung der beim Scanning zu durchlaufenden Rasterpunkte 62 für jede Schicht 22 überprüfen (siehe Fig. 11 b). Dabei werden durch die Auswerteeinheit 56 vorzugsweise die x-Werte und y-Werte für alle Rasterpunkte 62 einer Schicht 22 vorgegebener Eindringtiefe abgeleitet, aus diesen Informationen der Verlauf der zweidimensionalen Anordnung der Rasterpunkte 62 in der Reihenfolge der Applikation ermittelt und dieser Verlauf auf vorbestimmte Kriterien überprüft, beispielsweise auf das Vorliegen von "Inselpunkten" oder Abweichungen in der Bestrahlungssystematik. Die Auswerteeinheit 56 leitet dabei die x-Werte und die y-Werte für alle Rasterpunkte 62 vorzugsweise aus den Magnetströmen der Ablenkmagnete 18, 20 der Ablenkeinheit 16 ab.

In einer weiterführenden Ausführungsform berechnet die Auswerteeinheit 56 aus der Anzahl und Energie der auf jeden Rasterpunkt 62 zu applizierenden geladenen Teilchen eine Raster-Dosisverteilung und überprüft diese nach vorgegebenen Kriterien auf ihre therapiespezifische Plausibilität. Dieser Ansatz erfordert eine enorm hohe Rechenleistung.

Besonders bevorzugt ist eine weitere Überprüfungsform, nach der alle vom Therapiekontrollsystem 42 an das 3D-Scanning-System 12 gelieferten Bestrahlungsdaten und Bestrahlungsbefehle 52 für eine Bestrahlungssitzung in der Auswerteeinheit 56 gespeichert werden und am nächsten Tag mit den gesamten dann erneut vom Therapiekontrollsystem 42 an das 3D-Scanning-System 12 gelieferten Bestrahlungsdaten und Bestrahlungsbefehlen 52 für die nächste Bestrahlungssitzung verglichen werden. Da die Therapiepläne üblicherweise vollkommen identische Bestrahlungspläne für sämtliche Sitzungen vorsehen, können auf diese Weise besonders einfach Abweichungen festgestellt werden, die in erster Linie aufgrund geänderter äußerer Einflüsse wie Temperatur, Luftdruck, Konfigurationsparameter der Anlage usw. auftreten können.

Bei allen Varianten kann die abschließende Auswertung der von der Auswerteeinheit 56 ermittelten Daten entweder durch einen klinischen Anwender manuell erfolgen oder automatisch über einen Computer.

Bei Anwendung mindestens eines, vorzugsweise aber mehrerer der oben genannten Überprüfungsverfahren ist es möglich, neben den bereits bestehenden besonders ausgeprägten Sicherheitseinrichtungen 34, 48 (einschließlich der Sensoren für den Behandlungsstrahl) eine weitere Instanz zu instrumentalisieren, welche die vom Therapiekontrollsystem 42 an das 3D-Scanning-System 12 gelieferten Bestrahlungsdaten und Bestrahlungsbefehle 52 für jeden spezifischen Anwendungsfall auf therapiespezifische Plausibilität hin überprüft und somit sogar zufällig auftretende Fehler in den Bestrahlungsdaten und Bestrahlungsbefehlen 52 unter Berücksichtigung der klinischen Relevanz nahezu vollständig ausschließt.

## Patentansprüche

1. Anlage zur Bestrahlung von Patienten mit geladenen Teilchen, mit
einer Raster-Scanning-Bestrahlungseinheit (1), die einen Teilchenbeschleuniger (2), eine Strahlführungseinheit (4) und ein 3D-Scanning-System (12) aufweist, wobei das 3D-Scanning-System (12) eine Energievariationseinheit (14) zur Einstellung der Energie des Teilchenstrahls und damit der Eindringtiefe des Strahls in den Patienten in Strahlrichtung und eine Ablenkeinheit (16) mit mehreren Ablenkmagneten (18, 20) zum zweidimensionalen Ablenken des Strahls zwischen einzelnen Rasterpunkten in jeder durch die Energievariationseinheit (14) definierten, quer zur Strahlrichtung angeordneten Schicht (22) vorgegebener Eindringtiefe im Patienten aufweist,
einem Therapieplanungssystem (24) zum Erzeugen von Therapieplanungsdaten (40), welche die aus der gewünschten Dosisverteilung abgeleitete Energie und Anzahl von geladenen Teilchen pro Rasterpunkt (62) in jeder Schicht (22) umfassen,
einem Therapiekontrollsystem (42), das die vom Therapieplanungssystem (24) erzeugten Therapieplanungsdaten (40) in Bestrahlungsdaten und Bestrahlungsbefehle (44, 52) für den Teilchenbeschleuniger (2), die Strahlführungseinheit (4) und das 3D-Scanning-System (12) umwandelt, und
einer Mehrzahl von Sicherheitseinrichtungen (34, 48, 56) zum Überprüfen der korrekten Umsetzung der Therapieplanungsdaten (40) und der Funktionalität der Anlage, wobei die Mehrzahl von Sicherheitseinrichtungen (34, 48, 56) eine Auswerteeinheit (56) umfasst, die die vom Therapiekontrollsystem (42) an das 3D-Scanning-System (12) gelieferten Bestrahlungsdaten und Bestrahlungsbefehle (52) überprüft,
**dadurch gekennzeichnet, dass**
die Auswerteeinheit (56) geeignet ist, die vom Therapiekontrollsystem (42) an das 3D-Scanning-System (12) gelieferten Bestrahlungsdaten und Bestrahlungsbefehle (52) auf ihre therapiespezifische Plausibilität zu überprüfen, indem
a) die Auswerteeinheit (56) die vom Therapiekontrollsystem (42) erzeugten Bestrahlungsdaten und Bestrahlungsbefehle (52) auf ihre therapiespezifische Plausibilität hinsichtlich erlaubter Energiebereiche der geladenen Teilchen überprüft; und/oder indem
b) die Raster-Scanning-Bestrahlungseinheit (1) ein drehbares Gantry (10) aufweist und die Auswerteeinheit (56) die vom Therapiekontrollsystem (42) erzeugten Bestrahlungsdaten und Bestrahlungsbefehle (52) auf ihre therapiespezifische Plausibilität hinsichtlich erlaubter Gantry-Winkel für die Applikation der geladenen Teilchen überprüft; und/oder indem
c) die Auswerteeinheit (56) die vom Therapiekontrollsystem (42) erzeugten Bestrahlungsdaten und Bestrahlungsbefehle (52) auf ihre therapiespezifische Plausibilität hinsichtlich der Anzahl von zu applizierenden geladenen Teilchen pro Rasterpunkt (62) überprüft; und/oder indem
d) die Auswerteeinheit (56) die vom Therapiekontrollsystem (42) erzeugten Bestrahlungsdaten und Bestrahlungsbefehle (52) auf ihre therapiespezifische Plausibilität hinsichtlich vorbestimmter logischer Ordnungsprinzipien untersucht, wobei
d1) die Auswerteeinheit (56) die streng abnehmende Monotonität der Energiewerte der zu applizierenden geladenen Teilchen zur Überprüfung der abnehmenden Eindringtiefe zwischen den einzelnen beim Scanning zu durchlaufenden Schichten (22) überprüft; und/oder wobei
d2) die Auswerteeinheit (56) die Reihenfolge und Anordnung der beim Scanning zu durchlaufenden Rasterpunkte (62) für jede Schicht (22) überprüft.

2. Anlage nach Anspruch 1, **dadurch gekennzeichnet, dass** das 3D-Scanning-System (12) ein Scanning-Steuermodul (60) aufweist, welches dazu geeignet ist, die vom Therapiekontrollsystem (42) versandten Bestrahlungsdaten und Bestrahlungsbefehle (52) entgegenzunehmen.

3. Anlage nach Anspruch 2, **dadurch gekennzeichnet, dass** die Auswerteeinheit (56) zwischen Therapiekontrollsystem (42) und Scanning-Steuermodul (60) oder im Scanning-Steuermodul (60) angeordnet ist.

4. Anlage nach einem der vorangehenden Ansprüche, **dadurch gekennzeichnet, dass** die Auswerteeinheit (56) geeignet ist, eine vom Therapiekontrollsystem (42) erzeugte Datei mit Bestrahlungsdaten und Bestrahlungsbefehlen (52) zu untersuchen und eine Nachricht (58) über die Untersuchung zu erzeugen.

5. Verfahren zur Überwachung einer Anlage zur Bestrahlung von Patienten mit geladenen Teilchen, mit folgenden Schritten:
- Bereitstellen einer Raster-Scanning-Bestrahlungseinheit (1), die einen Teilchenbeschleuniger (2), eine Strahlführungseinheit (4) und ein 3D-Scanning-System (12) aufweist, wobei das 3D-Scanning-System (12) eine Energievariationseinheit (14) zur Einstellung der Energie des Teilchenstrahls und damit der Eindringtiefe des Strahls in den Patienten in Strahlrichtung und eine Ablenkeinheit (16) mit mehreren Ablenkmagneten (18, 20) zum zweidimensionalen Ablenken des Strahls zwischen einzelnen Rasterpunkten (62) in jeder durch die Energievariationseinheit (14) definierten, quer zur Strahlrichtung angeordneten Schicht (22) vorgegebener Eindringtiefe im Patienten aufweist,
- Erzeugen von Therapieplanungsdaten (40) in einem Therapieplanungssystem (24), wobei die Therapieplanungsdaten (40) die aus der gewünschten Dosisverteilung abgeleitete Energie und Anzahl von geladenen Teilchen pro Rasterpunkt (62) in jeder Schicht (22) umfassen,
- Umwandeln der vom Therapieplanungssystem (24) erzeugten Therapieplanungsdaten (40) in Bestrahlungsdaten und Bestrahlungsbefehle (44, 52) für den Teilchenbeschleuniger (2), die Strahlführungseinheit (4) und das 3D-Scanning-System (12) mittels eines Therapiekontrollsystems (42), und
- Überprüfen der korrekten Umsetzung der Therapieplanungsdaten (40) und der Funktionalität der Anlage mittels einer Mehrzahl von. Sicherheitseinrichtungen (34, 48, 56), wobei das Überprüfen der korrekten Umsetzung der Therapieplanungsdaten (40) und der Funktionalität der Anlage das Überprüfen der vom Therapiekontrollsystem (42) an das 3D-Scanning-System (12) gelieferten Bestrahlungsdaten und Bestrahlungsbefehle (52) mittels einer Auswerteeinheit (56) umfasst,
**dadurch gekennzeichnet, dass**
die vom Therapiekontrollsystem (42) an das 3D-Scanning-System (12) gelieferten Bestrahlungsdaten und Bestrahlungsbefehle (52) mittels der Auswerteeinheit (56) auf ihre therapiespezifische Plausibilität geprüft werden, indem
a) die Auswerteeinheit (56) die vom Therapiekontrollsystem (42) erzeugten Bestrahlungsdaten und Bestrahlungsbefehle (52) auf ihre therapiespezifische Plausibilität hinsichtlich erlaubter Energiebereiche der geladenen Teilchen überprüft; und/oder indem
b) die Raster-Scanning-Bestrahlungseinheit (1) ein drehbares Gantry (10) aufweist und die Auswerteeinheit (56) die vom Therapiekontrollsystem (42) erzeugten Bestrahlungsdaten und Bestrahlungsbefehle (52) auf ihre therapiespezifische Plausibilität hinsichtlich erlaubter Gantry-Winkel für die Applikation der geladenen Teilchen überprüft; und/oder indem
c) die Auswerteeinheit (56) die vom Therapiekontrollsystem (42) erzeugten Bestrahlungsdaten und Bestrahlungsbefehle (52) auf ihre therapiespezifische Plausibilität hinsichtlich der Anzahl von zu applizierenden geladenen Teilchen pro Rasterpunkt (62) überprüft; und/oder indem
d) die Auswerteeinheit (56) die vom Therapiekontrollsystem (42) erzeugten Bestrahlungsdaten und Bestrahlungsbefehle (52) auf ihre therapiespezifische Plausibilität hinsichtlich vorbestimmter logischer Ordnungsprinzipien untersucht, wobei
d1) die Auswerteeinheit (56) die streng abnehmende Monotonität der Energiewerte der zu applizierenden geladenen Teilchen zur Überprüfung der abnehmenden Eindringtiefe zwischen den einzelnen beim Scanning zu durchlaufenden Schichten (22) überprüft; und/oder wobei
d2) die Auswerteeinheit (56) die Reihenfolge und Anordnung der beim Scanning zu durchlaufenden Rasterpunkte (62) für jede Schicht (22) überprüft.

6. Verfahren nach Anspruch 5, **dadurch gekennzeichnet, dass** die Auswerteeinheit (56), welche entweder in einem Scanning-Steuermodul (60) oder zwischen Therapiekontrollsystem (42) und dem Scanning-Steuermodul (60) angeordnet ist, eine vom Therapiekontrollsystem (42) erzeugte Datei mit Bestrahlungsdaten und Bestrahlungsbefehlen (52) auf ihre therapiespezifische Plausibilität untersucht und eine Nachricht (58) über das Ergebnis der Untersuchung erzeugt.

7. Verfahren nach Anspruch 5 oder 6 enthaltend Schritt d2), **dadurch gekennzeichnet, dass** die Auswerteeinheit die x-Werte und y-Werte für alle Rasterpunkte (62) einer Schicht (22) vorgegebener Eindringtiefe ableitet, daraus den Verlauf der zweidimensionalen Anordnung der Rasterpunkte (62) in der Reihenfolge der Applikation ermittelt und diesen Verlauf auf vorbestimmte Kriterien überprüft.

8. Verfahren nach Anspruch 7, **dadurch gekennzeichnet, dass** die Auswerteeinheit (56) die x-Werte und y-Werte für alle Rasterpunkte (62) aus den Magnetströmen der Ablenkmagnete (18, 20) der Ablenkeinheit (16) ableitet.

9. Verfahren nach einem der Ansprüche 5 bis 8, **dadurch gekennzeichnet, dass** alle vom Therapiekontrollsystem (42) an das 3D-Scanning-System (12) gelieferten Bestrahlungsdaten und Bestrahlungsbefehle (52) für eine Bestrahlungssitzung in der Auswerteeinheit (56) gespeichert werden und mit den gesamten dann erneut vom Therapiekontrollsystem (42) an das 30-Scanning-System (12) gelieferten Bestrahlungsdaten und Bestrahlungsbefehlen (52) für die nächste Bestrahlungssitzung verglichen werden.

## Claims

1. Assembly for irradiating patients with charged particles, having
a raster scanning - irradiation unit (1) which has a particle accelerator (2), a beam guiding unit (4) and a 3D scanning system (12), wherein the 3D scanning system (12) has an energy variation unit (14) to adjust the energy of the particle beam and thus the penetration depth of the beam in the patient in the beam direction and a deflection unit (16) having a plurality of deflecting magnets (18, 20) for two-dimensional deflection of the beam between individual raster points in each layer (22), defined by the energy variation unit (14) and arranged transversely to the beam direction, of predetermined penetration depth in the patient,
a treatment planning system (24) for generating treatment planning data (40) which comprise the energy derived from the desired dose distribution and number of charged particles for each raster point (62) in each layer (22),
a treatment control system (42) which converts the treatment planning data (40) generated by the treatment planning system (24) into irradiation data and irradiation commands (44, 52) for the particle accelerator (2), the beam guiding unit (4) and the 3D scanning system (12), and
a plurality of safety means (34, 48, 56) to check the correct implementation of the treatment planning data (40) and the functionality of the assembly, wherein the plurality of safety means (34, 48, 56) comprise an evaluation unit (56) which checks the irradiation data and irradiation commands (52) supplied by the treatment control system (42) to the 3D scanning system (12),
**characterised in that**
the evaluation unit (56) is suited to check the irradiation data and irradiation commands (52) supplied by the treatment control system (42) to the 3D scanning system (12) for their treatment-specific plausibility, **in that**
a) the evaluation unit (56) checks the irradiation data and irradiation commands (52) generated by the treatment control system (42) for their treatment-specific plausibility with regard to permitted energy ranges of the charged particles; and / or **in that**
b) the raster scanning - irradiation unit (1) has a rotating gantry (10) and the evaluation unit (56) checks the irradiation data and irradiation commands (52) generated by the treatment control system (42) for their treatment-specific plausibility with regard to permitted gantry angles for the application of the charged particles; and / or **in that**
c) the evaluation unit (56) checks the irradiation data and irradiation commands (52) generated by the treatment control system (42) for their treatment-specific plausibility with regard to the number of charged particles to be applied for each raster point (62); and / or **in that**
d) the evaluation unit (56) examines the irradiation data and irradiation commands (52) generated by the treatment control system (42) for their treatment-specific plausibility in relation to predetermined logical ordering principles, wherein
dl) the evaluation unit (56) checks the strictly decreasing monotony of the energy values of the charged particles to be applied to check the decreasing penetration depth between the individual layers (22) to be crossed during scanning; and / or wherein
d2) the evaluation unit (56) checks the sequence and arrangement of the raster points (62) for each layer (22) to be crossed during scanning.

2. Assembly according to claim 1, **characterised in that** the 3D scanning system (12) has a scanning control module (60) which is suited to receive the irradiation data and irradiation commands (52) sent by the treatment control system (42).

3. Assembly according to claim 2, **characterised in that** the evaluation unit (56) is arranged between the treatment control system (42) and the scanning control module (60) or in the scanning control module (60).

4. Assembly according to one of the preceding claims, **characterised in that** the evaluation unit (56) is suited to examine a file generated by the treatment control system (42) with irradiation data and irradiation commands (52) and to generate a report (58) on the examination.

5. Method for monitoring an assembly for irradiating patients with charged particles, having the following steps:
- provide a raster scanning - irradiation unit (1) which has a particle accelerator (2), a beam guiding unit (4) and a 3D scanning system (12), wherein the 3D scanning system (12) has an energy variation unit (14) to adjust the energy of the particle beam and thus the penetration depth of the beam in the patient in the beam direction and a deflection unit (16) having a plurality of deflecting magnets (18, 20) for two-dimensional deflection of the beam between individual raster points (62) in each layer (22), defined by the energy variation unit (14) and arranged transversely to the beam direction, of predetermined penetration depth in the patient,
- generate treatment planning data (40) in a treatment planning system (24), wherein the treatment planning data (40) comprise the energy and number of charged particles for each raster point (62) in each layer (22) derived from the desired dose distribution,
- convert the treatment planning data (40) generated by the treatment planning system (24) into irradiation data and irradiation commands (44, 52) for the particle accelerator (2), the beam guiding unit (4) and the 3D scanning system (12) by means of a treatment control system (42), and
- check the concrete implementation of the treatment planning data (40) and the functionality of the assembly by means of a plurality of safety means (34, 48, 56), wherein the checking of the concrete implementation of the treatment planning data (40) and the functionality of the assembly comprises the checking of the irradiation data and irradiation commands (52) supplied by the treatment control system (42) to the 3D scanning system (12) by means of an evaluation unit (56),
**characterised in that**
the irradiation data and irradiation commands (52) supplied by the treatment control system (42) to the 3D scanning system (12) are checked by means of the evaluation unit (56) for their treatment-specific plausibility, **in that**
a) the evaluation unit (56) checks the irradiation data and irradiation commands (52) generated by the treatment control system (42) for their treatment-specific plausibility in relation to permitted energy ranges of the charged particles; and / or **in that**
b) the raster scanning - irradiation unit (1) has a rotating gantry (10) and the evaluation unit (56) checks the irradiation data and irradiation commands (52) generated by the treatment control system (42) for their treatment-specific plausibility in relation to permitted gantry angles for the application of the charged particles; and / or **in that**
c) the evaluation unit (56) checks the irradiation data and irradiation commands (52) generated by the treatment control system (42) for their treatment-specific plausibility in relation to the number of charged particles to be applied for each raster point (62); and / or **in that**
d) the evaluation unit (56) examines the irradiation data and irradiation commands (52) generated by the treatment control system (42) for their treatment-specific plausibility in relation to predetermined logical ordering principles, wherein
d1) the evaluation unit (56) checks the strictly decreasing monotony of the energy values of the charged particles to be applied for the purpose of checking the decreasing penetration depth between the individual layers (22) to be crossed during scanning; and / or wherein
d2) the evaluation unit (56) checks the sequence and arrangement of the raster points (62) for each layer (22) to be crossed during scanning.

6. Method according to claim 5, **characterised in that** the evaluation unit (56), which is arranged either in a scanning control module (60) or between the treatment control system (42) and the scanning control module (60), examines a file generated by the treatment control system (42) with irradiation data and irradiation commands (52) for their treatment-specific plausibility and generates a report (58) on the result of the examination.

7. Method according to claim 5 or 6, containing step d2), **characterised in that** the evaluation unit derives the x values and y values for all raster points (62) of a layer (22) of predetermined penetration depth, determining from these the pattern of the two-dimensional arrangement of the raster points (62) in the sequence of the application, and checking this pattern for predetermined criteria.

8. Method according to claim 7, **characterised in that** the evaluation unit (56) derives the x values and y values for all raster points (62) from the magnetic flows of the deflecting magnets (18, 20) of the deflection unit (16).

9. Method according to one of claims 5 to 8, **characterised in that** all irradiation data and irradiation commands (52) supplied by the treatment control system (42) to the 3D scanning system (12) for an irradiation session are stored in the evaluation unit (56) and are compared with all the irradiation data and irradiation commands (52) then supplied again by the treatment control system (42) to the 3D scanning system (12) for the next irradiation session.

## Revendications

1. Installation d'irradiation de patients avec des particules chargées, comprenant
une unité d'irradiation à balayage tramé (1), qui comprend un accélérateur de particules (2), une unité de guidage de faisceau (4) et un système de balayage 3D (12), le système de balayage 3D (12) comprenant une unité de variation d'énergie (14) permettant d'ajuster l'énergie du faisceau de particules et donc la profondeur de pénétration du faisceau dans le patient dans la direction d'irradiation et une unité de déviation (16) comprenant plusieurs aimants de déviation (18, 20) permettant la déviation bidimensionnelle du faisceau entre différents points de trame dans chaque couche (22), d'une profondeur de pénétration prédéfinie dans le patient, définie par l'unité de variation d'énergie (14) et disposée transversalement à la direction d'irradiation,
un système de planification de thérapie (24) permettant de produire des données de planification de thérapie (40), lesquelles incluent l'énergie déduite de la répartition de la dose souhaitée et le nombre de particules chargées par point de trame (62) dans chaque couche (22),
un système de contrôle de thérapie (42), qui convertit les données de planification de thérapie (40) produites par le système de planification de thérapie (24) en données d'irradiation et en instructions d'irradiation (44, 52) pour l'accélérateur de particules (2), l'unité de guidage de faisceau (4) et le système de balayage 3D (12), et
une pluralité de dispositifs de sécurité (34, 48, 56) permettant de procéder à la vérification de la conversion correcte des données de planification de thérapie (40) et de la fonctionnalité de l'installation, la pluralité de dispositifs de sécurité (34, 48, 56) comportant une unité d'évaluation (56) qui procède à la vérification des données d'irradiation et des instructions d'irradiation (52) délivrées par le système de contrôle de thérapie (42) au système de balayage 3D (12),
**caractérisée en ce que**
l'unité d'évaluation (56) est adaptée à procéder à la vérification de la plausibilité spécifique à la thérapie des données d'irradiation et des instructions d'irradiation (52) délivrées par le système de contrôle de thérapie (42) au système de balayage 3D (12), par le fait que
a) l'unité d'évaluation (56) procède à la vérification de la plausibilité spécifique à la thérapie des données d'irradiation et des instructions d'irradiation (52) produites par le système de contrôle de thérapie (42) en ce qui concerne les plages d'énergie autorisées des particules chargées ; et/ou par le fait que
b) l'unité d'irradiation à balayage tramé (1) comprend un portique rotatif (10) et l'unité d'évaluation (56) procède à la vérification de la plausibilité spécifique à la thérapie des données d'irradiation et des instructions d'irradiation (52) produites par le système de contrôle de thérapie (42) en ce qui concerne les angles autorisés du portique pour l'application des particules chargées ; et/ou par le fait que
c) l'unité d'évaluation (56) procède à la vérification de la plausibilité spécifique à la thérapie des données d'irradiation et des instructions d'irradiation (52) produites par le système de contrôle de thérapie (42) en ce qui concerne le nombre de particules chargées à appliquer par point de trame (62) ; et/ou par le fait que
d) l'unité d'évaluation (56) analyse la plausibilité spécifique à la thérapie des données d'irradiation et des instructions d'irradiation (52) produites par le système de contrôle de thérapie (42) en ce qui concerne les principes d'ordre logiques prédéfinis,
d1) l'unité d'évaluation (56) procédant à la vérification de la monotonie fortement décroissante des valeurs énergétiques des particules chargées à appliquer pour vérifier la profondeur de pénétration décroissante entre les différentes couches (22) destinées à être traversées lors du balayage ; et/ou
d2) l'unité d'évaluation (56) procédant à la vérification de l'ordre et de la disposition des points de trame (62) destinés à être traversés lors du balayage pour chaque couche (22).

2. Installation selon la revendication 1, **caractérisée en ce que** le système de balayage 3D (12) comprend un module de commande de balayage (60), lequel est adapté à recevoir les données d'irradiation et les instructions d'irradiation (52) envoyées par le système de contrôle de thérapie (42).

3. Installation selon la revendication 2, **caractérisée en ce que** l'unité d'évaluation (56) est disposée entre le système de contrôle de thérapie (42) et le module de commande de balayage (60) ou dans le module de commande de balayage (60).

4. Installation selon l'une quelconque des revendications précédentes, **caractérisée en ce que** l'unité d'évaluation (56) est adaptée à analyser un fichier produit par le système de contrôle de thérapie (42) et comprenant les données d'irradiation et les instructions d'irradiation (52), et à produire un message (58) concernant l'analyse.

5. Procédé de vérification d'une installation d'irradiation de patients avec des particules chargées, comprenant les étapes suivantes :
la fourniture d'une unité d'irradiation à balayage tramé (1), qui comprend un accélérateur de particules (2), une unité de guidage de faisceau (4) et un système de balayage 3D (12), le système de balayage 3D (12) comprenant une unité de variation d'énergie (14) permettant d'ajuster l'énergie du faisceau de particules et donc la profondeur de pénétration du faisceau dans le patient dans la direction d'irradiation et une unité de déviation (16) comprenant plusieurs aimants de déviation (18, 20) permettant la déviation bidimensionnelle du faisceau entre différents points de trame (62) dans chaque couche (22), d'une profondeur de pénétration prédéfinie dans le patient, définie par l'unité de variation d'énergie (14) et disposée transversalement à la direction d'irradiation,
la production de données de planification de thérapie (40) dans un système de planification de thérapie (24), les données de planification de thérapie (40) incluant l'énergie déduite de la répartition de la dose souhaitée et le nombre de particules chargées par point de trame (62) dans chaque couche (22),
la conversion des données de planification de thérapie (40) produites par le système de planification de thérapie (24) en données d'irradiation et en instructions d'irradiation (44, 52) pour l'accélérateur de particules (2), l'unité de guidage de faisceau (4) et le système de balayage 3D (12) au moyen d'un système de contrôle de thérapie (42), et
la vérification de la conversion correcte des données de planification de thérapie (40) et de la fonctionnalité de l'installation au moyen d'une pluralité de dispositifs de sécurité (34, 48, 56), la vérification de la conversion correcte des données de planification de thérapie (40) et de la fonctionnalité de l'installation incluant la vérification des données d'irradiation et des instructions d'irradiation (52) délivrées par le système de contrôle de thérapie (42) au système de balayage 3D (12) au moyen d'une unité d'évaluation (56),
**caractérisée en ce que**
on procède à la vérification de la plausibilité spécifique à la thérapie des données d'irradiation et des instructions d'irradiation (52) délivrées par le système de contrôle de thérapie (42) au système de balayage 3D (12) au moyen de l'unité d'évaluation (56), par le fait que
a) l'unité d'évaluation (56) procède à la vérification de la plausibilité spécifique à la thérapie des données d'irradiation et des instructions d'irradiation (52) produites par le système de contrôle de thérapie (42) en ce qui concerne les plages d'énergie autorisées des particules chargées ; et/ou par le fait que
b) l'unité d'irradiation à balayage tramé (1) comprend un portique rotatif (10) et l'unité d'évaluation (56) procède à la vérification de la plausibilité spécifique à la thérapie des données d'irradiation et des instructions d'irradiation (52) produites par le système de contrôle de thérapie (42) en ce qui concerne les angles autorisés du portique pour l'application des particules chargées ; et/ou par le fait que
c) l'unité d'évaluation (56) procède à la vérification de la plausibilité spécifique à la thérapie des données d'irradiation et des instructions d'irradiation (52) produites par le système de contrôle de thérapie (42) en ce qui concerne le nombre de particules chargées à appliquer par point de trame (62) ; et/ou par le fait que
d) l'unité d'évaluation (56) analyse la plausibilité spécifique à la thérapie des données d'irradiation et des instructions d'irradiation (52) produites par le système de contrôle de thérapie (42) en ce qui concerne les principes d'ordre logiques prédéfinis,
d1) l'unité d'évaluation (56) procédant à la vérification de la monotonie fortement décroissante des valeurs énergétiques des particules chargées à appliquer pour contrôler la profondeur de pénétration décroissante entre les différentes couches (22) destinées à être traversées lors du balayage ; et/ou
d2) l'unité d'évaluation (56) procédant à la vérification de la succession et de la disposition des points de trame (62) destinés à être traversés lors du balayage pour chaque couche (22).

6. Procédé selon la revendication 5, **caractérisé en ce que** l'unité d'évaluation (56), laquelle est disposée soit dans un module de commande de balayage (60) ou entre le système de contrôle de thérapie (42) et le module de commande de balayage (60), analyse un fichier produit par le système de contrôle de thérapie (42) comprenant des données d'irradiation et des instructions d'irradiation (52) et produit un message (58) concernant le résultat de l'analyse.

7. Procédé selon la revendication 5 ou 6 contenant l'étape d2), **caractérisé en ce que** l'unité d'évaluation déduit les valeurs x et les valeurs y pour tous les points de trame (62) d'une couche (22) d'une profondeur de pénétration prédéfinie, détermine à partir de ces celles-ci le tracé de la disposition bidimensionnelle des points de trame (62) dans l'ordre de l'application et procède à la vérification des critères prédéfinis de ce tracé.

8. Procédé selon la revendication 7, **caractérisé en ce que** l'unité d'évaluation (56) déduit les valeurs x et les valeurs y pour tous les points de trame (62) des flux magnétiques des aimants de déviation (18, 20) de l'unité de déviation (16).

9. Procédé selon l'une quelconque des revendications 5 à 8, **caractérisé en ce que** toutes les données d'irradiation et instructions d'irradiation (52) délivrées par le système de contrôle de thérapie (42) au système de balayage 3D (12) pour une séance d'irradiation sont mises en mémoire dans l'unité d'évaluation (56) et comparées à l'ensemble des données d'irradiation et des instructions d'irradiation (52) à nouveau délivrées par le système de contrôle de thérapie (42) au système de balayage 3D (12) pour la prochaine séance d'irradiation.
